# EUROPEAN PATENT SPECIFICATION

(11) **EP 4 298 183 B1**
(45) Date of publication and mention of the grant of the patent: **04.06.2025**
(21) Application number: 22707879.7
(22) Date of filing: 24.02.2022
(51) Int. Cl.: C09K 5/10, H01M 10/613, H01M 10/6567

(54) **DIELECTRIC THERMAL MANAGEMENT FLUIDS AND METHODS FOR USING THEM**
DIELEKTRISCHE WÄRMEVERWALTUNGSFLÜSSIGKEITEN UND VERFAHREN ZU IHRER VERWENDUNG
FLUIDES DIÉLECTRIQUES DE GESTION THERMIQUE ET LEURS PROCÉDÉS D'UTILISATION

(30) Priority: 24.02.2021 US 202163153163 P
(43) Date of publication of application: 03.01.2024
(73) Proprietor: Castrol Limited, Reading RG8 7QR (GB)
(72) Inventor: GARRETT, James, Houston, Texas 77079 (US); DEELEY, Jon, Reading, RG8 7QR (GB); ARMITAGE, Gareth, Reading, RG8 7QR (GB); WEST, Kevin, Reading, RG8 7QR (GB); FILIP, Sorin, Reading, RG8 7QR (GB); PRENTICE, Giles, Reading, RG8 7QR (GB)
(74) Representative: Hamer, Christopher K.
(86) International application number: PCT/IB2022/051610
(87) International publication number: WO 2022/180553

(56) References cited:
- WO-A1-2020/216690
- JP-A- 2005 084 074
- US-A- 2 973 388

## Description

### CROSS-REFERENCE TO RELATED APPLICATIONS

This application claims the benefit of priority of U.S. Provisional Patent Application no. 63/153163, filed February 24, 2021.

### BACKGROUND OF THE DISCLOSURE

### Field of the Disclosure

This disclosure relates generally to thermal management fluids. More particularly this disclosure relates to a dielectric thermal management fluid suitable for use managing heat in battery systems through direct cooling, such as lithium-ion batteries used in electric vehicles, electric motors, and power electronics, methods of using such thermal management fluids, and systems including such thermal management systems.

### Technical Background

The number of electric vehicles (i.e., vehicles using electric power for all or a portion of their motive power such as battery electric vehicles (BEVs), hybrid electric vehicles (HEVs), plug-in hybrid electric vehicles (PHEVs), and the like) sold globally has increased over the last several years, and is expected to continue to increase. Ultimately, the vast majority of vehicles will likely be electric. As electric vehicle technology continues to evolve, there is a need to provide improved power sources (e.g., battery systems or modules). For example, it is desirable to increase the distance that such vehicles may travel without the need to recharge the batteries, to improve the performance of such batteries, and to reduce the costs and time associated with battery charging.

Most batteries will generate heat as current is delivered to or drawn from the batteries. Typically, as the amount of current flowing into or out of the battery increases, the amount of heat that is generated also increases. If the heat that is generated is not dissipated the battery will rise in temperature. Most batteries have an effective operating temperature range, and if the battery exceeds the maximum operating temperature, the batteries can become ineffective or even result in thermal runaway and failure. In some cases, after a slight rise in temperature, a battery may be able to dissipate heat to its surroundings through a simple heat sink or without any thermal management. In other cases, a more specific thermal management system is needed to dissipate heat that is generated by the battery.

Currently, battery-powered electric vehicles almost exclusively use lithium-ion battery technology. Lithium-ion batteries offer many advantages over the comparable nickel-metal-hydride batteries, but as compared to nickel-metal-hydride batteries, lithium-ion batteries are more susceptible to variations in battery temperature and thus have more stringent thermal management requirements. For example, optimal lithium-ion battery operating temperatures are in the range of 10 and 35 °C. Operation is increasingly inefficient as temperatures rise from 35 to 70 °C, and, more critically, operation at these temperatures can damage the battery over time. Temperatures over 70 °C present increased risk of thermal runaway. As a result, lithium-ion batteries require specific thermal management systems to regulate their temperatures during vehicle operation. In addition, during charging, up to 10% of the inputted power ends up as heat. As the fast charging of lithium-ion batteries becomes more common, the need remains for efficient systems for thermal management of the batteries.

Lithium-ion batteries may be cooled directly or indirectly, using thermal management fluids to carry heat away from the battery component (i.e., as a cooling fluid or coolant). Direct cooling advantageously allows the thermal management fluid to come into direct contact with the hot components to carry heat away therefrom. In indirect cooling, a hot component is electrically shielded by an electrically-insulating barrier and the thermal management fluid carries away heat passing through this barrier. The most common thermal management fluids are based on mixtures of water with glycol. But because water-based fluids typically conduct electricity, they cannot be used in the direct cooling of electrical components of lithium-ion batteries. While indirect cooling allows for water-based coolants to be used, the requirement of electrical shielding can create a bottleneck for heat flow in the cooling process. There exist dielectric thermal management fluids that can be used for direct cooling of electrical components due to their non-electrically-conductive nature; examples include those conventionally used in the cooling of electrical transformers. However, the thermal properties of such dielectric thermal management fluids are typically poor in comparison to water-glycol. WO 2020/216690 discloses a thermal management fluid comprising a dielectric fluid which comprises one or more dielectric substances, the dielectric fluid having a flash point of less than 150°C measured in accordance with ASTM D93 and a dielectric constant of at least 1.5 at 25°C, the dielectric fluid being present in a total amount in the range of 75wt% to 99.9wt%. The thermal management fluid further comprises one or more halocarbons each having a boiling point in the range of 60°C to 200°C, present in a total amount in the range of 0.1wt% to 20wt%, and homogeneously dispersed in the dielectric thermal management fluid. The thermal management fluid does not have a flash point of less than 120°C, measured in accordance with ASTM D93, and has a dielectric constant of at least 1.5 at 25°C.

Thus, there remains a need for improved dielectric thermal management systems, especially those suitable for use in the cooling of lithium-ion batteries.

### SUMMARY OF THE DISCLOSURE

The present invention is as defined in the appended claims. Disclosed herein but not encompassed with this the scope of the present claims are thermal management fluids that have a flash point of at least 100 °C, measured in accordance with ASTM D93, and have a dielectric constant of at least 1.5 at 25 °C. Such dielectric thermal management fluids include:
one or more dielectric compounds of formula (I): 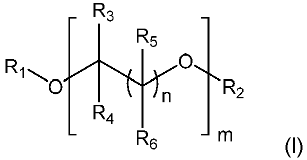 wherein
m is an integer 1, 2, 3, 4 or 5;
n is an integer 1, 2, 3, 4 or 5;
R₁ is C₁-C₅ alkyl;
R₂ is C₆-C₁₂ alkyl;
each R₃ and R₄ are independently selected from H and C₁-C₆ alkyl; and
each R₅ and R₆ are independently selected from H and C₁-C₆ alkyl;
the one or more dielectric compounds being present in a total amount in the range of 1 wt% to 100 wt%, based on the total weight of the thermal management fluid.

In accordance with a first aspect of the present invention, there is provided a battery system. The battery system comprises a housing; one or more electrochemical cells disposed in the housing; a fluid path extending in the housing and in substantial thermal communication with the one or more electrochemical cells; and a thermal management fluid of the disclosure as described herein disposed in the fluid path.

In some examples, an electric vehicle may comprise the battery system of the disclosure as described herein.

In accordance with a second aspect of the present invention, there is provided a thermal management circuit comprising: a fluid path extending around and/or through a heat source; and a thermal management fluid of the disclosure, disposed in and configured to circulate in the fluid path and to absorb thermal energy produced by the heat source, wherein the fluid is disposed in the fluid path, the heat exchanger, the pump and the connecting duct.

In accordance with a third aspect of the present invention, there is provided a method comprising contacting a thermal management fluid of the disclosure with a surface having a temperature of at least 25 °C (e.g., at least 30 °C), the surface being in substantial thermal communication with a heat source; and absorbing thermal energy in the thermal management fluid from the heat source through the surface.

In accordance with a fourth aspect of the present invention, there is provided a thermal management fluid comprising: one or more dielectric compounds of the formula 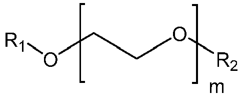 wherein
m is an integer 1, 2, or 3;
R₁ is a methyl, ethyl, propyl, or butyl, or
R₁ is isopropyl, t-butyl, sec-butyl, or 1,1-dimethylpropyl; and

R₂ is a group -CH₂-CH(R_{b})(R_{d}) in which R_{b} is methyl or ethyl, and R_{d} is C₃-C₈ alkyl, e.g., R₂ is 2-ethylhexyl;
the one or more dielectric compounds being present in a total amount in the range of 1 wt% to 100 wt%, based on the total weight of the thermal management fluid; and
wherein the thermal management fluid has a flash point of at least 100 °C, measured in accordance with ASTM D93, and the thermal management fluid has a dielectric constant of at least 1.5 at 25 °C.

Disclosed herein but not encompassed within the scope of the present claims is a method for preparing the thermal management fluid of the disclosure. Such method includes contacting a compound of formula (II) 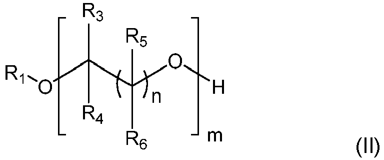 wherein
m is an integer 1, 2, 3, 4 or 5;
n is an integer 1, 2, 3, 4 or 5;
R₁ is C₁-C₅ alkyl; and
each R₃, R₄, R₅ and R₆ are independently selected from H and C₁-C₆ alkyl,
with (C₆-C₁₂ alkyl)-L, wherein L is a leaving group to obtain a dielectric compound of formula (I).

Also disclosed herein but not encompassed within the scope of the present claims is a method for preparing the thermal management fluid of the disclosure. Such method includes contacting a compound of formula (III) 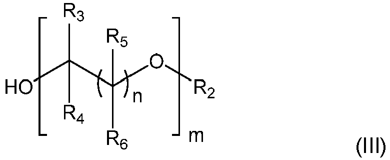 wherein
m is an integer 1, 2, 3, 4 or 5;
n is an integer 1, 2, 3, 4 or 5;
R₂ is C₆-C₁₂ alkyl; and
each R₃, R₄, R₅ and R₆ are independently selected from H and C₁-C₆ alkyl,
with (C₁-C₅ alkyl)-L, wherein L is a leaving group to obtain a dielectric compound of formula (I).

### BRIEF DESCRIPTION OF THE DRAWINGS

The accompanying drawings are included to provide a further understanding of the compositions and methods of the disclosure, and are incorporated in and constitute a part of this specification. The drawings are not necessarily to scale, and sizes of various elements may be distorted for clarity. The drawings illustrate one or more embodiment(s) of the disclosure and, together with the description, serve to explain the principles and operation of the disclosure.
FIG. 1 is a schematic cross-sectional view of a thermal management circuit according to an embodiment of the disclosure.
FIG. 2 is a schematic cross-sectional view of a thermal management circuit according to another embodiment of the disclosure.

### DETAILED DESCRIPTION

The present inventors have noted that desirable thermal management fluids would in many cases have a high capacity to carry heat away in a temperature range relevant to operation of a particular electrical device or system (e.g., a lithium-ion battery), yet have a sufficiently high dielectric constant to be suitable for use in direct cooling of the device or system. Critically, because there is always a risk that oxygen might enter the overall system, desirable thermal management fluids would advantageously have a high flash point, to reduce the risk of ignition. And to provide more efficient heat transfer during the operation, desirable thermal management fluids would advantageously have low viscosity allowing for better flowability in a particular electrical device or system.

The present inventors have identified thermal management fluid compositions that provide not only a desirably low viscosity but also have a high flash point, so they can be easily pumped through a system with low-to-no risk of ignition. Specifically, the present inventors recognized that conventional dielectric fluids (e.g., organic or silicone) typically have good thermal conductivity and specific heat capacity but have undesirably high viscosity. Typical low-viscosity dielectric fluids, however, generally have unacceptably low flash points (and other ignition properties) making them unsuitable for use as coolants in systems where there is the potential for temperatures to rise where ignition is a risk. The present inventors have determined that the dielectric compounds of the disclosure can provide a thermal management fluid that does not have a low flash point and has advantageously low viscosity. These properties of the thermal management fluid make them particularly suitable, for example, for direct cooling of electrical devices and systems.

The thermal management fluids and methods of the disclosure can have a number of additional advantages over conventional fluids. Notably, the thermal management fluid of the disclosure can also, in various embodiments, provide one or more of desirably high heat conductivity, low risk of ignition, high dielectric constant, and fast temperature response. The thermal management fluid of the disclosure can in various embodiments also have lower surface tension than conventional low-viscosity dielectric fluids.

Thus, disclosed herein but not encompassed within the scope of the present claims is a thermal management fluid including one or more dielectric compounds of formula (I), the one or more dielectric compounds being present in a total amount in the range of 1 wt% to 100 wt%. Such thermal management fluids can have a flash point of at least 100 °C, measured in accordance with ASTM D93 ("Standard Test Methods for Flash Point by Pensky-Martens Closed Cup Tester"), and a dielectric constant of at least 1.5 at 25 °C.

Because there is always some risk that oxygen might enter the system, the thermal management fluids of the disclosure advantageously have a high flash point to prevent ignition. As described above, the thermal management fluids of the disclosure have a flash point of at least 100 °C, as measured in accordance with ASTM D93. For example, in various embodiments, a thermal management fluid as otherwise described herein has a flash point of at least 110 °C, e.g., at least 120 °C, at least 125 °C, at least 130 °C, or at least 135 °C, measured in accordance with ASTM D93. In various embodiments, a thermal management fluid as otherwise described herein has a flash point of at least 140 °C, e.g., at least 145 °C, at least 150 °C, or at least 155 °C, measured in accordance with ASTM D93. A material that does not have a flash point below 100 °C is considered to have a flash point above 100 °C for the purposes of this disclosure, even if no flash point is measurable for the material (i.e., due to decomposition at a temperature below where a flash point is reached).

A low viscosity is often desired for a thermal management fluid, to simplify the pumping thereof through a system, especially when relatively narrow passageways are used. The person of ordinary skill in the art will, based on the present disclosure, select components to provide the thermal management fluids with a desired viscosity, e.g., to be conveniently conducted through a system. Accordingly, in various embodiments, a thermal management fluid as otherwise described herein has a kinematic viscosity at 40 °C in the range of 0.0000015 to 0.00002 m²/s (1.5 to 20 cSt), e.g., in the range of 0.0000015 to 0.000015 m²/s (1.5 to 15 cSt), or 0.000002 to 0.000015 m²/s (2 to 15 cSt), or 0.000002 to 0.00002 m²/s (2 to 20 cSt), or 0.000003 to 0.00002 m²/s (3 to 20 cSt), or 0.000003 to 0.000015 m²/s (3 to 15 cSt), or 0.000005 to 0.00002 m²/s (5 to 20 cSt), or 0.000005 to 0.000015 m²/s (5 to 15 cSt). In various embodiments, a thermal management fluids as otherwise described herein has a kinematic viscosity at 40 °C in the range of 0.0000015 to 0.00001 m²/s (1.5 to 10 cSt), e.g., 0.0000015 to 0.000008 m²/s (1.5 to 8 cSt), or 0.0000015 to 0.000006 m²/s (1.5 to 6 cSt), or 0.000002 to 0.00001 m²/s (2 to 10 cSt), or 0.000002 to 0.000008 m²/s (2 to 8 cSt), or 0.000002 to 0.000006 m²/s (2 to 6 cSt), or 0.000003 to 0.00001 m²/s (3 to 10 cSt), or 0.000003 to 0.000008 m²/s (3 to 8 cSt), or 0.000003 to 0.000006 m²/s (3 to 6 cSt), or 0.000005 to 0.00001 m²/s (5 to 10 cSt), or 0.000005 to 0.000008 m²/s (5 to 8 cSt), or 0.000005 to 0.000006 m²/s (5 to 6 cSt), or 0.000006 to 0.00001 m²/s (6 to 10 cSt), or 0.000008 to 0.00001 m²/s (8 to 10 cSt), as measured in accordance with ASTM D455. And in various embodiments, a thermal management fluids as otherwise described herein has a kinematic viscosity at 40 °C in the range of 0.0000015 to 0.000005 m²/s (1.5 to 5 cSt), e.g., 0.0000015 to 0.000004 m²/s (1.5 to 4 cSt), or 0.0000015 to 0.000003 m²/s (1.5 to 3 cSt), or 0.000002 to 0.000005 m²/s (2 to 5 cSt), or 0.000002 to 0.000004 m²/s (2 to 4 cSt), or 0.000002 to 0.000003 m²/s (2 to 3 cSt), or 0.000003 to 0.000005 m²/s (3 to 5 cSt), or 0.000003 to 0.000004 m²/s (3 to 4 cSt), or 0.000004 to 0.000005 m²/s (4 to 5 cSt), as measured in accordance with ASTM D455.

The thermal management fluids of the disclosure are dielectric, so that they can be used in direct cooling applications. Accordingly, they have a dielectric constant of at least 1.5 as measured at 25 °C. The dielectric constant is measured using the coaxial probe method, using ASTM D924. In various embodiments, a thermal management fluid of the disclosure has a dielectric constant of at least 1.75, e.g., at least 2.0, or at least 2.25 as measured at 25 °C. In various embodiments, a thermal management fluid of the disclosure has a dielectric constant in the range of 1.5 to 10, or 1.8 to 10, or 1.5 to 2.8, or 1.8 to 2.8.

In various embodiments of the disclosure, the thermal management fluid of the disclosure may have density of no more than 1.1 g/cm³ at 25 °C. For example, in various embodiments of the disclosure, the thermal management fluid of the disclosure may have density of no more than 1 g/cm³ at 25 °C.

In various embodiments of the disclosure, the thermal management fluid of the disclosure may have a heat capacity of at least 1 J/g·K, or at least 1.2 J/g·K, or even at least 1.5 J/g·K, at 25 °C. In various embodiments of the disclosure, the thermal management fluid of the disclosure may have a thermal conductivity in the range of 0.05 W/m·K to 1 W/m·K at 25 °C. In various embodiments of the disclosure, the thermal management fluid of the disclosure may have a coefficient of thermal expansion is no more than 1100 × 10⁻⁶/K (e.g., no more than 1050 × 10⁻⁶/K, or no more than 1000 × 10⁻⁶/K).

As described above, the thermal management fluid of the disclosure includes one or more dielectric compounds of formula (I).

The person of ordinary skill in the art can select the chain length and branching of R₁ and R₂ based on the disclosure herein to select properties of the overall material, e.g., viscosity and flash point.

In the one or more dielectric compounds of formula (I) as described herein R₁ is C₁-C₅ alkyl. In various embodiments as otherwise described herein, in one or more dielectric compounds of formula (I) R₁ is C₃-C₅ alkyl. In various embodiments as otherwise described herein, in one or more dielectric compounds of formula (I) R₁ is a branched C₃-C₅ alkyl, such as branched C₄-C₅ alkyl. Branching can be, for example, at an α-position or at a β-position with respect to the oxygen atom to which R₁ is bound. In various embodiments, branching is at an α-position with respect to the oxygen atom. For example, in various embodiments as otherwise described herein, in one or more dielectric compounds of formula (I) R₁ is an α-branched C₃-C₅ alkyl, such as t-butyl or t-pentyl. In various embodiments as otherwise described herein, in one or more dielectric compounds of formula (I) R₁ is -C(CH₃)(CH₃)(R_{c}), wherein R_{c} is methyl or ethyl.

In the one or more dielectric compounds of formula (I) as otherwise described herein R₂ is C₆-C₁₂ alkyl, such as C₆-C₁₀ or C₆-C₈ alkyl. In various embodiments of the dielectric compounds of formula (I) of the disclosure, R₂ is C₈-C₁₂ alkyl, such as C₈-C₁₀ alkyl or C₁₀-C₁₂ alkyl. In various embodiments of the dielectric compounds of formula (I) of the disclosure, R₂ is a branched C₆-C₁₂ alkyl, such as branched C₆-C₁₀ alkyl, branched C₆-C₈ alkyl, branched C₈-C₁₂ alkyl, branched C₈-C₁₀ alkyl, or branched C₁₀-C₁₂ alkyl. Branching can be, for example, at an α-position or at a β-position with respect to the oxygen atom to which R₂ is bound. In various embodiments, branching is at a β-position with respect to the oxygen atom. For example, in certain compounds of formula (I), R₂ is a β-branched C₆-C₁₂ alkyl, such as β-branched C₆-C₁₀ alkyl, β-branched C₆-C₈ alkyl, β-branched C₈-C₁₂ alkyl, β-branched C₈-C₁₀ alkyl, or β-branched C₁₀-C₁₂ alkyl.

In various embodiments, in the one or more dielectric compounds of formula (I) as described herein each of R₃, R₄, R₅ and R₆ is independently selected from H and C₁-C₄ alkyl (such as C₁-C₃ alkyl or C₁-C₂ alkyl). In certain other embodiments, each of R₃, R₄, R₅ and R₆ is independently selected from H and C₄ alkyl (i.e., methyl).

In various embodiments, each R₄ is H and each R₃ is H or C₁-C₆ alkyl (such as C₁-C₄ alkyl or C₁-C₂ alkyl); and each R₅ is H and each R₆ is H or C₁-C₆ alkyl (such as C₁-C₄ alkyl or C₁-C₂ alkyl). In various embodiments, R₃ is H, R₄ is H, R₅ is H and each R₆ is H or C₁-C₆ alkyl (such as C₁-C₄ alkyl or C₁-C₂ alkyl).

In various embodiments as otherwise described herein, in the one or more dielectric compounds of formula (I) each of R₃, R₄, R₅, and R₆ is H, i.e., the compounds have the formula:

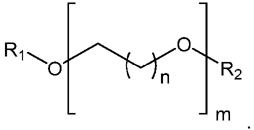

In various embodiments, in the one or more dielectric compounds of formula (I) as otherwise described herein, each of R₄, R₅, and R₆ is H, and R₃ is H or C₁-C₆ alkyl. For example, in some embodiments, each of R₄, R₅, and R₆ is H, and R₃ is C₁-C₆ alkyl (such C₁-C₄ alkyl, C₁-C₃ alkyl, ethyl, or methyl) , i.e., the compounds have the formula 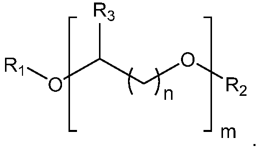 In various embodiments, each of R₄, R₅, and R₆ is independently H, and wherein R₃ is C₁-C₆ alkyl (such C₁-C₄ alkyl, C₁-C₃ alkyl, ethyl, or methyl).

In various embodiments, in the one or more dielectric compounds of formula (I) as otherwise described herein, n is an integer 1 or 2. In various embodiments, n is an integer 2 or 3. In various embodiments, n is an integer 1, i.e., the compounds have the formula:

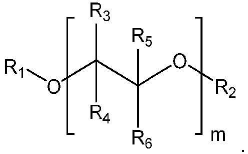

In various embodiments, the one or more dielectric compounds is of formula:

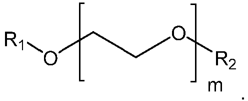

In various embodiments, the one or more dielectric compounds is of formula:

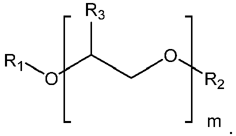

In various embodiments, n is an integer 2, i.e., the compounds have the formula:

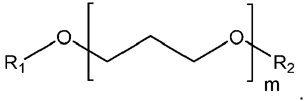

In various embodiments as otherwise described herein, in the one or more dielectric compounds of formula (I) m is 1. However, in other embodiments, m is 2, or m is 3. In other embodiments, m is 4, or is 5.

In various embodiments as otherwise described herein, in the one or more dielectric compounds of formula (I) has the formula 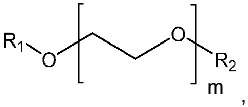 in which m is 1, 2, 3, 4, or 5, R₁ is C₁-C₅ alkyl, and R₂ is C₆-C₁₀ alkyl. In certain such embodiment, R₁ is methyl or ethyl. In certain such embodiments, R₁ is propyl (e.g., n-propyl, isopropyl) or butyl, e.g., n-butyl, t-butyl, sec-butyl or isobutyl. In certain such embodiments, R₁ is a branched pentyl, e.g., 1,1-dimethylpropyl, 2,2-dimethylpropyl. In certain such embodiments, R₂ is branched, e.g., 2-ethylhexyl, or a group -CH₂-CH(R_{b})(R_{d}) in which R_{b} is methyl or ethyl, and R_{d} is C₃-C₈ alkyl, such as 2-ethylhexyl. In certain such embodiments, m is 1, In other embodiments, m is or 2 or 3. In still other embodiments, m is 4 or 5.

In various embodiments as otherwise described herein, the one or more dielectric compounds of formula (I) contain a total number of carbon atoms from 10 to 30 (e.g., from 10 to 26, from 10 to 22, from 10 to 18, from 12 to 30, from 12 to 26, from 12 to 22, from 14 to 30, from 14 to 26, or from 14 to 22). For example, in various embodiments the one or more dielectric compounds of formula (I) contains a total number of carbon atoms from 12 to 18. In various embodiments the one or more dielectric compounds of formula (I) contains a total number of carbon atoms from 14 to 22. In various embodiments the one or more dielectric compounds of formula (I) contains a total number of carbon atoms from 14 to 20.

Examples of the compounds of formula (I) of the disclosure include, but are not limited to: 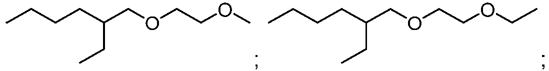 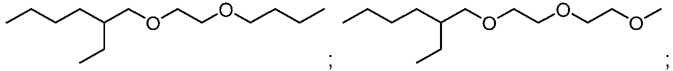 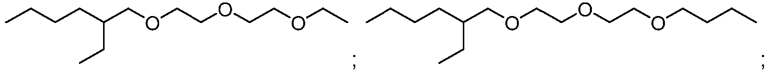 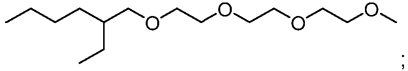 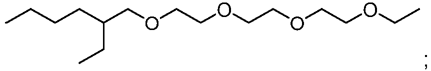 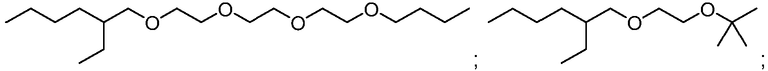 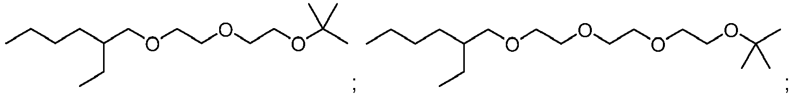 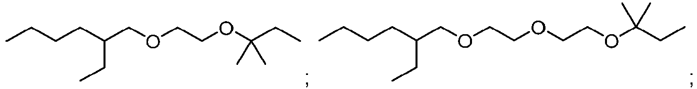 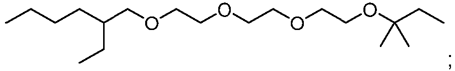 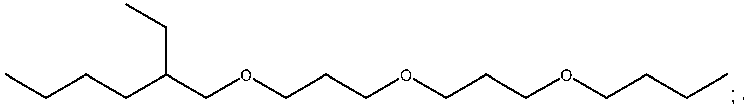 and

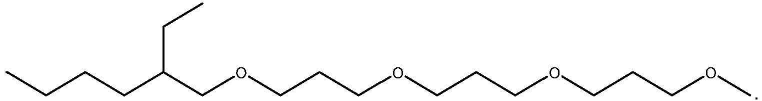

In various embodiments as otherwise described herein, the one or more dielectric compounds of the thermal management fluid have a flash point of at least 100 °C measured in accordance with ASTM D93. The present inventors have advantageously determined that the use of the dielectric compounds of the disclosure having high flash points can provide an overall thermal management fluid with a high flash point and thereby decrease the risk of ignition. In various embodiments of the thermal management fluids as otherwise described herein, the one or more dielectric compounds has a flash point of at least 110 °C (e.g., at least 120 °C, at least 125 °C, at least 130 °C, or at least 135 °C) or at least 140 °C (e.g., at least 145 °C, at least 150 °C, or at least 155 °C), measured in accordance with ASTM D93.

The present inventors have advantageously determined that the one or more dielectric compounds as described herein have relatively low viscosity yet a reduced risk of ignition. Accordingly, in various embodiments of the thermal management fluids as otherwise described herein, the one or more dielectric compounds has a kinematic viscosity at 40 °C in the range of 0.0000015 to 0.00002 m²/s (1.5 to 20 cSt), e.g., in the range of 0.0000015 to 0.000015 m²/s (1.5 to 15 cSt), or 0.000002 to 0.00002 m²/s (2 to 20 cSt), or 0.000002 to 0.000015 m²/s (2 to 15 cSt), or 0.000003 to 0.00002 m²/s (3 to 20 cSt), or 0.000003 to 0.000015 m²/s (3 to 15 cSt), or 0.000005 to 0.00002 m²/s (5 to 20 cSt), or 0.000005 to 0.000015 m²/s (5 to 15 cSt). In various embodiments of the thermal management fluids as otherwise described herein, the one or more dielectric compounds has a kinematic viscosity at 40 °C in the range of 0.0000015 to 0.00001 m²/s (1.5 to 10 cSt), e.g., 0.0000015 to 0.000008 m²/s (1.5 to 8 cSt), or 0.0000015 to 0.000006 m²/s (1.5 to 6 cSt), or 0.000002 to 0.00001 m²/s (2 to 10 cSt), or 0.000002 to 0.000008 m²/s (2 to 8 cSt), or 0.000002 to 0.000006 m²/s (2 to 6 cSt), or 0.000003 to 0.00001 m²/s (3 to 10 cSt), or 0.000003 to 0.000008 m²/s (3 to 8 cSt), or 0.000003 to 0.000006 m²/s (3 to 6 cSt), or 0.000005 to 0.00001 m²/s (5 to 10 cSt), or 0.000005 to 0.000008 m²/s (5 to 8 cSt), or 0.000005 to 0.000006 m²/s (5 to 6 cSt), or 0.000006 to 0.00001 m²/s (6 to 10 cSt), or 0.000008 to 0.00001 m²/s (8 to 10 cSt), as measured in accordance with ASTM D455. And In various embodiments of the thermal management fluids as otherwise described herein, the one or more dielectric compounds has a kinematic viscosity at 40 °C in the range of 0.0000015 to 0.000005 m²/s (1.5 to 5 cSt), or 0.0000015 to 0.000004 m²/s (1.5 to 4 cSt), or 0.0000015 to 0.000003 m²/s (1.5 to 3 cSt), or 0.000002 to 0.000005 m²/s (2 to 5 cSt), or 0.000002 to 0.000004 m²/s (2 to 4 cSt), or 0.000002 to 0.000003 m²/s (2 to 3 cSt), or 0.000003 to 0.000005 m²/s (3 to 5 cSt), or 0.000003 to 0.000004 m²/s (3 to 4 cSt), or 0.000004 to 0.000005 m²/s (4 to 5 cSt), as measured in accordance with ASTM D455.

The person of ordinary skill in the art will appreciate that various combinations of dielectric compounds of the disclosure can be used in thermal management fluids of the disclosure. Accordingly, embodiments of dielectric compounds described above can be combined in any number and in any combination in thermal management fluids of the disclosure. When two or more dielectric compounds are used in a thermal management fluid, the relative amounts of the two can be varied based on the disclosure herein, depending on the effect desired. In various embodiments, the mass ratio of a first dielectric compound to a second dielectric compound is in the range of 1:9 to 9:1 (e.g., 1:5 to 5:1, or 1:5 to 1:1, or 1:1 to 5:1).

The one or more dielectric compounds can be present in the thermal management fluids described herein in a variety of amounts. In various embodiments as otherwise described herein, the one or more dielectric compounds is present in a total amount in the range of 1 wt% to 100 wt% (e.g., 5 wt% to 100 wt%, or 10 wt% to 100 wt%, or 20 wt% to 100 wt%) based on the total weight of the thermal management fluid. For example, in various embodiments, the one or more dielectric compounds is present in a total amount in the range of 50 wt% to 100 wt%, for example, 75 wt% to 100 wt%, or 85 wt% to 100 wt%, or 90 wt% to 100 wt%, or 95 wt% to 100 wt%, or 98 wt% to 100 wt%. For example, in various embodiments of the thermal management fluid as otherwise described herein, the one or more dielectric compounds is present in a total amount in the range of 1 wt% to 99.9 wt% (e.g., 5 wt% to 99.9 wt%, or 10 wt% to 99.9 wt%, or 20 wt% to 99.9 wt%), or 50 wt% to 99.9 wt%, for example, 75 wt% to 99.9 wt%, or 85 wt% to 99.9 wt%, or 90 wt% to 99.9 wt%, or 95 wt% to 99.9 wt%, or 98 wt% to 99.9 wt%, based on the total weight of the thermal management fluid. In various embodiments of the thermal management fluid as otherwise described herein, the one or more dielectric compounds is present in a total amount in the range of 1 wt% to 99 wt% (e.g., 5 wt% to 99 wt%, or 10 wt% to 99 wt%, or 20 wt% to 99 wt%), or 50 wt% to 99 wt%, for example, 75 wt% to 99 wt%, or 85 wt% to 99 wt%, or 90 wt% to 99 wt%, or 95 wt% to 99 wt%, based on the total weight of the thermal management fluid. In various embodiments of the thermal management fluid as otherwise described herein, the one or more dielectric compounds is present in a total amount in the range of 1 wt% to 95 wt% (e.g., 5 wt% to 95 wt%, or 10 wt% to 95 wt%, or 20 wt% to 95 wt%), or 50 wt% to 95 wt%, for example, 75 wt% to 95 wt%, or 85 wt% to 95 wt%, based on the total weight of the thermal management fluid. In various embodiments of the thermal management fluid as otherwise described herein, the one or more dielectric compounds is present in a total amount in the range of 1 wt% to 85 wt% (e.g., 5 wt% to 85 wt%, or 10 wt% to 85 wt%, or 20 wt% to 85 wt%), or 50 wt% to 85 wt%, for example, 65 wt% to 85 wt%, or 75 wt% to 85 wt%, based on the total weight of the thermal management fluid. The person of ordinary skill in the art will, based on the disclosure herein, provide the dielectric compound(s) in an amount to provide a desired high flash point to the thermal management fluid, in addition with any other desired properties (e.g., viscosity).

As the person of ordinary skill in the art will appreciate based, the thermal management fluids of the disclosure can also include a variety of other components, such as those conventional in compositions for thermal management applications. For example, the thermal management fluid may further include an oil, e.g., a mineral oil, a synthetic oil, or a silicone oil. For example, in various embodiments, the oil is a low-viscosity Group II, III, IV, or V base oil as defined by the American Petroleum Institute (API Publication 1509). These are shown in Table 1.

**Table 1 - Base Oil Stocks API Guidelines**

| | **Saturates** | **Sulfur content** | **Viscosity Index (VI)** |
|---|---|---|---|
| **Group I** | <90 and/or | >300 ppm and | ≥80 and <120 |
| **Group II** | ≥90 and | ≤300 ppm and | ≥80 and <120 |
| **Group III** | ≥90 and | ≤300 ppm and | ≥120 |
| **Group IV** | Includes polyalphaolefins (PAO) and GTL (gas-to-liquid) products | | |
| **Group V** | All other base oils not included in Groups I, II, III or IV | | |

Group II and Group III base oils (such as hydrocracked and hydroprocessed base oils as well as synthetic oils such as hydrocarbon oils, polyalphaolefins, alkyl aromatics, and synthetic esters) and Group IV base oils (such as polyalphaolefins (PAO)) are wells known base oils. Oils suitable for use as transformer oils can, in many embodiments, be suitable for use in the compositions, systems and methods of the disclosure. For example, esters also form a useful base oil stock, including synthetic esters, as do GTL (gas-to-liquid) materials, particularly those derived from a hydrocarbon source. For example, the esters of dibasic acids with monoalcohols, or the polyol esters of monocarboxylic acid may be useful as base stocks of the disclosure. Bio-derived oils such as fatty acid methyl esters may also be useful.

In various embodiments, the thermal management fluid of the disclosure further comprises a Group II, Group III, Group IV, or Group V base oil. For example, in various embodiments, the thermal management fluid of the disclosure further comprises a Group II or Group III base oil. In certain other embodiments, the thermal management fluid of the disclosure further comprises a Group IV base oil such as polyalphaolefins (PAO). In certain other embodiments, the thermal management fluid of the disclosure further comprises an ester base oil stock.

In various embodiments, the thermal management fluid of the disclosure further comprises one or more of corrosion inhibitors, anti-oxidants (such as phenolic and aminic anti-oxidants), pour point depressants, antifoams, defoamers, viscosity index modifiers, preservatives, biocides, surfactants, seal swell additives, and combinations thereof. In various embodiments, corrosion inhibitors, anti-oxidants (such as phenolic and aminic anti-oxidants), pour point depressants, antifoams, defoamers, viscosity index modifiers, preservatives, biocides, surfactants, seal swell additives, and combinations thereof, for example, may be present in an amount up to 5.0 wt%, based on the total weight of the thermal management fluid. In certain such embodiments, one or more of corrosion inhibitors, anti-oxidants (such as phenolic and aminic anti-oxidants), pour point depressants, antifoams, defoamers, viscosity index modifiers, preservatives, biocides, surfactants, seal swell additives, and combinations thereof are present in an amount in the range of 0.2 wt% to 5.0 wt%, e.g., 1.0 wt% to 2.0 wt%, or 0.2 wt% to 1.0 wt%, or 0.2 wt% to 0.5 wt%, or 0.05 wt% to 0.2 wt%, based on the total weight of the thermal management fluid. In various embodiments, the thermal management fluid of the disclosure further comprises one or more flame retardants, e.g., in an amount up to 20 wt%, up to 10 wt%, or up to 5 wt%, based on the total weight of the thermal management fluid. In other embodiments, however, no flame retardant is present.

Another aspect of the disclosure provides a method comprising contacting a thermal management fluid as described herein with a surface having a temperature of at least 25 °C, the surface being in substantial thermal communication with a heat source, and absorbing thermal energy in the thermal management fluid from the heat source through the surface.

The contacting of the thermal management fluid with the surface can be dynamic or static (i.e., conductive). For example, in various embodiments, the contacting of the thermal management fluid with the surface can be performed by circulating, e.g., by pumping or otherwise flowing, the fluid over the surface. In various embodiments, the contacting can also be performed without circulation, e.g., by contacting the surface with the thermal management fluid that is a stationary body of fluid.

The temperature of the surface can vary; the thermal management fluid can be adapted for use with a variety of temperatures. In various embodiments as otherwise described herein, the temperature of the surface is in the range of 25 °C to 150 °C, e.g., 25 °C to 100 °C, or 25 °C to 90 °C, or 25 °C to 85 °C, or 25 °C to 80 °C, or 25 °C to 75 °C, or 25 °C to 70 °C. In various embodiments as otherwise described herein, the temperature of the surface is in the range of 30 °C to 150 °C, e.g., 30 °C to 100 °C, or 30 °C to 90 °C, or 30 °C to 85 °C, or 30 °C to 80 °C, or 30 °C to 75 °C, or 30 °C to 70 °C. In various embodiments as otherwise described herein, the temperature of the surface is in the range of 40 °C to 150 °C, e.g., 50 °C to 150 °C, or 60 °C to 150 °C, or 70 °C to 150 °C, or 80 °C to 150 °C, or 90 °C to 150 °C, or 100 °C to 150 °C, or 110 °C to 150 °C. The temperature of the surface in various embodiments (and at certain times during operation of a device or system) is no more than a boiling point of any of the one or more dielectric compounds of formula (I) of the thermal management system. In various embodiments, throughout the contacting, each of the one or more dielectric compounds of formula (I) does not reach its boiling point.

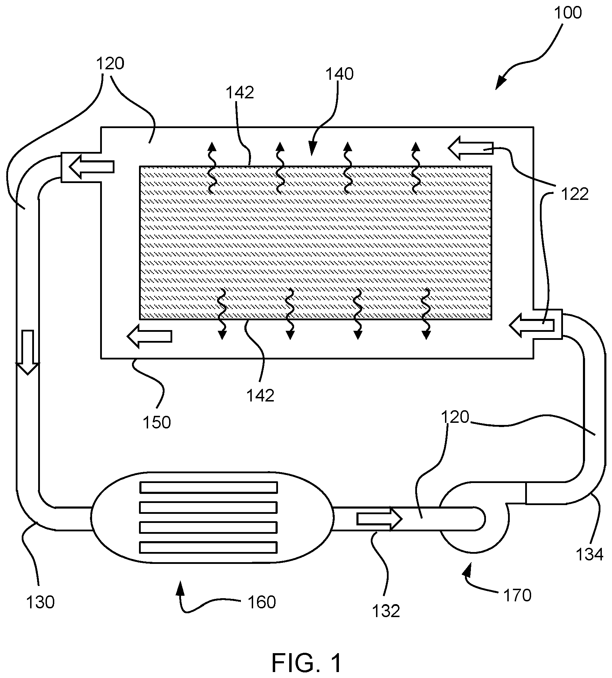

An embodiment of the method of the disclosure is illustrated with reference to FIG. 1. A thermal management circuit 100 is shown in a schematic cross-sectional side view in FIG. 1. The thermal management circuit 100 includes a thermal management fluid 120 that is circulated through the circuit and passes over surface 142. The temperature of surface 142 is elevated in comparison to the temperature of thermal management fluid 120. As a result, thermal energy is absorbed in thermal management fluid 120 from surface 142.

In various embodiments as otherwise described herein, the method includes producing the thermal energy by operating an electrical component. For example, thermal management circuit 100 is associated with electrical component 140, which produces heat during operation. In various embodiments the heat is produced as elements of the electrical component charge and discharge. As will be understood by those of ordinary skill in the art, inefficiencies in the operation of the electrical component and resistances in the circuits corresponding circuits create heat as current passes through the circuits and elements of the electrical component. For example, the heat from the operation of electrical component 140 causes surface 142 to rise in temperature, which then results in the transfer of thermal energy to thermal management fluid 120. In other embodiments, the thermal energy is produced by a chemical reaction, such as an exothermic reaction, or by friction. In still other embodiments, the thermal management fluid is chilled and absorbs thermal energy from surfaces at ambient or slightly elevated temperatures.

In various embodiments as otherwise described herein, the electrical component includes a battery system, a capacitor, inverter, electrical cabling, a fuel cell, a motor, or a computer. For example, in various embodiments the electrical component is a battery system that includes one or more electrochemical cells disposed in a housing. In other embodiments the electrical component is one or more capacitors, such as an electrolytic capacitor or an electric double-layer capacitor, e.g., a supercapacitor. In still other embodiments, the electrical component is one or more fuel cells, such as a polymer electrolyte membrane fuel cell, a direct methanol fuel cell, an alkaline fuel cell, a phosphoric acid fuel cell, a molten carbonate fuel cell, a solid oxide fuel cell, or a reversible fuel cell. In various embodiments the electrical component is an electric motor. In other embodiments, the electrical component is a computer, for example a personal computer or a server. Still, in other embodiments, the electrical component is a high power charging equipment.

The electrical component of the disclosure can operate on direct current (DC) or alternating current (AC). In various embodiments as otherwise described herein, the electrical component operates at DC or AC voltage above 48 V. In various embodiments as otherwise described herein, the electrical component operates at DC or AC voltage above 100 V, above 200 V, or above 300 V.

In various embodiments as otherwise described herein, the surface is a surface of the electrical component. For example, in FIG. 1 a housing of 150 of electrical component 140 contains a reservoir of thermal management fluid 120. Elements of the electrical component including certain circuits that produce heat is submerged in thermal management fluid 120 and the thermal management fluid absorbs thermal energy directly from an outside surface 142 of the electrical component 140.

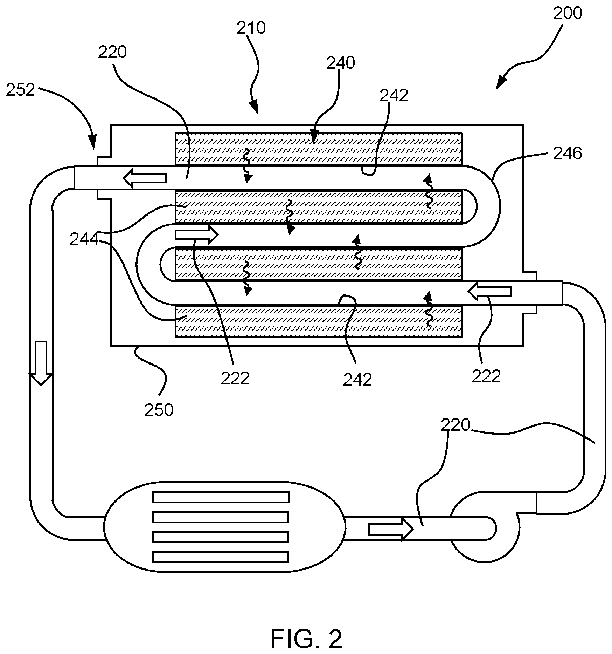

In various embodiments as otherwise described herein, the surface is an internal surface of a conduit. For example, FIG. 2 shows a thermal management circuit 200 that includes electrical component 240 that includes a plurality of individual units 244. In particular, the electrical component 240 is a battery that includes a plurality of electrochemical cells 244. Electrical component 240 further includes a conduit 246 that extends through the inside of the electrical component and between the electrochemical cells 244. As the electrical component produces thermal energy, the internal surface 242 of the conduit 246 is heated and the thermal energy is absorbed by the thermal management fluid 220.

In various embodiments as otherwise described herein, the conduit passes through a housing that surrounds the electrical component. For example, conduit 246 in thermal management circuit 200 extends through apertures 252 in the housing 250 surrounding electrical component 240, which allow thermal management fluid 220 to be conveyed to other elements of the thermal management circuit 200.

Another aspect of the disclosure provides a battery system including: a housing; one or more electrochemical cells disposed in the housing; a fluid path extending through the housing and in substantial thermal communication with the one or more electrochemical cells; and a thermal management fluid according to any of the embodiments described above that is disposed in the fluid path. For example, thermal management circuit 200 in FIG. 2 includes battery system 210. The battery system includes a plurality of electrochemical cells 244 that are disposed inside housing 250. A conduit 246 forms a fluid path that extends through the housing. Thermal management fluid 220 disposed in conduit 246 is thereby placed in thermal communication with the electrochemical cells 244. As the electrochemical cells 244 charge and discharge they produce heat which is absorbed by the thermal management fluid 220. In various embodiments the electrochemical cells are subject to fast charging which yields a large amount of heat. The high heat capacity of the thermal management fluid is able to absorb this large amount of heat quickly as it is produced.

In various embodiments as otherwise described herein, the fluid path is at least partially defined by a cavity of the housing. For example, in various embodiments at least a portion of the fluid path is formed between the electrochemical cells and the inside wall of the housing, similar to fluid path 122 in component 140.

In various embodiments as otherwise described herein, the fluid path is at least partially defined by at least one conduit disposed in the housing. For example, in battery system 210, conduit 246 provides the fluid path 222 through the housing 250.

In various embodiments as otherwise described herein, the electrochemical cells are lithium-ion electrochemical cells. In other embodiments, the electrochemical cells are solid state cells, lithium-sulfur cells, lithium iron phosphate cells, lithium-ion polymer cells, sodium-ion cells, aluminum-ion cells, lead-acid cells, or magnesium-ion cells.

In various embodiments as otherwise described herein, the battery system is a component of an electric vehicle. In some embodiments, the electric vehicle is a fully electric vehicle or a hybrid electric vehicle. In other embodiments the battery system is component of a power motor, for example an electric motor or a motor in power electronics. In other embodiments the battery system is part of a stationary energy storage solution, for example a home energy storage solution that operates in cooperation with local renewable energy sources, such as solar panels or wind turbines.

Another aspect of the disclosure provides a thermal management circuit including a fluid path extending around and/or through a heat source; a thermal management fluid of according to any of embodiments described above, disposed in and configured to circulate in the fluid path and to absorb thermal energy produced by the heat source, wherein the fluid is disposed in the fluid path, the heat exchanger, the pump and the connecting duct. For example, thermal management circuit 100 shown in FIG. 1 includes a fluid path 122 that runs around electrical component 140. Thermal management fluid 120 flows through path 122 absorbing thermal energy from electronic component 140. From fluid path 122, the thermal management fluid 120 flows through a first duct 130 to heat exchanger 160. Thermal energy that has accumulated in thermal management fluid 120 is removed from the fluid within heat exchanger 160 before the fluid flows through a second duct 132 to pump 170. After pump 170, the thermal management fluid 120 passes through a third duct 134 returning it to fluid path 122 surrounding electrical component 140. Circuit 100, shown in FIG. 1, is a schematic depiction of an uncomplicated embodiment employing the described thermal management fluid. In other embodiments, the thermal management circuit includes additional elements, such as any combination of valves, pumps, heat exchangers, reservoirs and ducts.

In various embodiments of the as otherwise described herein, the heat source is a battery including a plurality of electrochemical cells, and wherein the fluid path passes between at least two of the electrochemical cells.

In various embodiments as otherwise described herein, the fluid path is defined by a housing around the electrical component. For example, housing 150 in FIG. 1 surrounds electrical component 140 and provides a cavity for thermal management fluid 120. Electrical component 140 is held in the housing at a distance from the walls of housing 150, which allows a path for thermal management fluid 120 to form between the housing 150 and the electrical component 140. While housing 150 has an enclosed shape with specific apertures 152 providing access for thermal management fluid 120, in other embodiments the top of the housing is open and the thermal management fluid is retained in the housing by gravity.

In various embodiments as otherwise described herein, the fluid path is configured to position the thermal management fluid in substantial thermal communication with the electrical component so as to absorb thermal energy produced by the electrical component. For example, in thermal management circuit 100 fluid path 122 extends around electrical component 140 and is in direct contact with the surfaces of electrical component 140. Further, in thermal management circuit 200 fluid path 222 passes through a conduit 246 that runs adjacent to the elements of electrical component 240. In both cases, the fluid path places thermal management fluid in close proximity to the electrical component so that the thermal management fluid readily absorbs thermal energy from the component.

In various embodiments as otherwise described herein, the thermal management circuit further includes a heat exchanger in fluid communication with the fluid path, wherein the thermal management fluid is configured to circulate between the fluid path and the heat exchanger to dissipate heat through the heat exchanger. In various embodiments as otherwise described herein, the heat exchanger is configured to remove heat from the thermal management fluid. For example, in thermal management circuit 100, after thermal management fluid 120 is pumped out of housing 150 it passes to heat exchanger 160 where the thermal energy is transferred to a cooler fluid, such as ambient air or a cooling liquid.

In various embodiments as otherwise described herein, the thermal management circuit includes a battery system according to any of the embodiments described above. For example, thermal management circuit 200 includes battery system 210. In various embodiments as otherwise described herein, the thermal management circuit includes an immobilized desiccant material disposed according to any of the embodiments described above. For example, thermal management circuit 300 includes battery desiccant material 360.

The particulars shown herein are by way of example and for purposes of illustrative discussion of various embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the invention (especially in the context of the following embodiments and claims) are to be construed to cover both the singular and the plural, unless otherwise indicated herein or clearly contradicted by context.

The term "alkyl" as used herein, means a straight or branched chain hydrocarbon containing from 1 to 12 carbon atoms unless otherwise specified. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl. When an "alkyl" group is a linking group between two other moieties, then it may also be a straight or branched chain; examples include, but are not limited to -CH₂-, -CH₂CH₂-, -CH₂CH₂CHC(CH₃)-, and-CH₂CH(CH₂CH₃)CH₂-.

All methods described herein can be performed in any suitable order of steps unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

### EXAMPLE

The methods of the disclosure are illustrated further by the following example.

### Preparation of dielectric compounds of formula (I)

The compounds of the disclosure can be easily prepared from inexpensive starting materials. For example, compounds of formula (I) can be made by contacting a compound of formula (II) 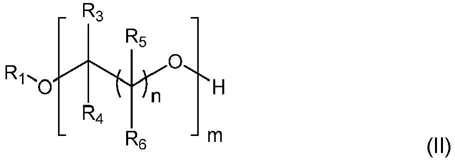 wherein
m is an integer 1, 2, 3, 4 or 5;
n is an integer 1, 2, 3, 4 or 5;
R₁ is C₁-C₅ alkyl; and
each R₃, R₄, R₅ and R₆ are independently selected from H and C₁-C₆ alkyl,
with (C₆-C₁₂ alkyl)-L, wherein L is a leaving group (e.g., bromide, , trifluoromethylsulfonate), optionally in the presence of a base (e.g., sodium hydroxide) to obtain a dielectric compound of formula (I).

Compounds of formula (I) can similarly be made by contacting a compound of formula (III) 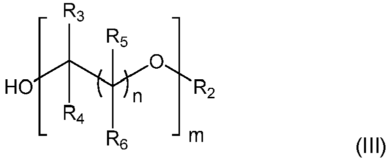 wherein
m is an integer 1, 2, 3, 4 or 5;
n is an integer 1, 2, 3, 4 or 5;
R₂ is C₆-C₁₂ alkyl; and
each R₃, R₄, R₅ and R₆ are independently selected from H and C₁-C₆ alkyl,
with (C₁-C₅ alkyl)-L, wherein L is a leaving group (e.g., bromide, trifluoromethanesulfonate), optionally in the presence of a base, to obtain a dielectric compound of formula (I).

Compounds of formula (I) can also be made by contacting a compound of formula (II) 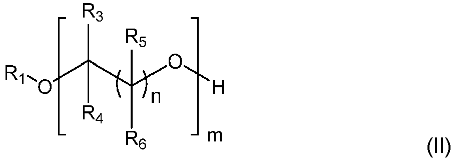 wherein
m is an integer 1, 2, 3, 4 or 5;
n is an integer 1, 2, 3, 4 or 5;
R₁ is C₁-C₅ alkyl; and
each R₃, R₄, R₅ and R₆ are independently selected from H and C₁-C₆ alkyl,
with a compound having the structure (C₆-C₁₂ alkyl-O)₃P=O in the presence of a Lewis acid catalyst (e.g., iron(II) trifluoroacetate) to obtain a dielectric compound of formula (I).

Compounds of formula (I) can also be made by contacting a compound of formula (III) 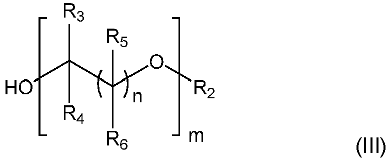 wherein
m is an integer 1, 2, 3, 4 or 5;
n is an integer 1, 2, 3, 4 or 5;
R₂ is C₆-C₁₂ alkyl; and
each R₃, R₄, R₅ and R₆ are independently selected from H and C₁-C₆ alkyl,
with (C₁-C₅ alkyl-O)₃P=O in the presence of a Lewis acid catalyst (e.g., iron(II) trifluoroacetate) to obtain a dielectric compound of formula (I).

The person of ordinary skill in the art will recognize that a variety of Lewis acids can be used in effecting these transformations. While iron(II) trifluoroacetate is a desirable Lewis acid, others can be used.

The ethers of formula (I) can generally be made by any convenient route -- for example, from commercially available alcohols, bromides, carboxylic acids, esters and aldehydes by Williamson ether synthesis, reduction of esters, hydrogenation of acetals or enol ethers. Methods described in International Patent Application Publication no. 2018/150185 can also be used; in such methods, an alcohol and an alkene are contacted with one another in the presence of an acidic ion-exchange resin to provide a corresponding ether. Methods described in International Patent Application no. 2020/104768 can also be used; in such methods, an aldehyde and a alcohol are combined with a catalyst such as pyridium p-toluenesulfonate with removal of water (e.g., using Dean-Stark techniques) to provide an enol ether, which is hydrogenated over platinum catalyst to provide the desired ether. Purification can be by any known method e.g. flash chromatography or distillation, with distillation being preferred industrially.

A compound of the Formula (I) has been synthesized according to the scheme below.

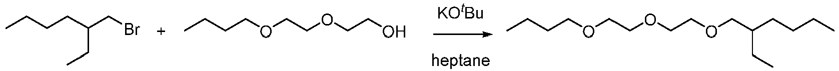

Alternatively, the compound can be prepared according to the scheme below: 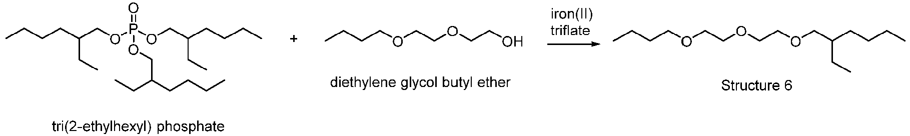 The alcohol is reacted with tri(2-ethylhexyl) phosphate (4.5 eq) in presence of iron(II)triflate (0.04eq) at 100 °C for 72 hrs. Upon completion, the reaction mixture is cooled to ambient temperature and partitioned between aqueous K₂CO₃ and hexane. The hexane layer is separated, dried over sodium sulphate and evaporated to give a yellow oil, which is distilled to provide the target ether.

Alternatively, the compound can be prepared according to the scheme below: 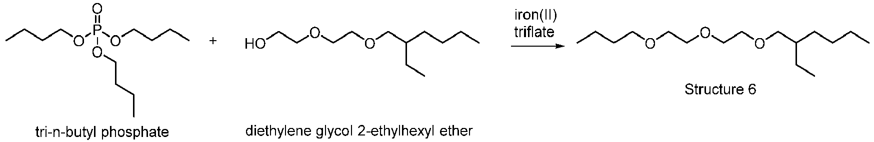 **The** alcohol is reacted with tri-n-butyl-phosphate (4.5 eq) in presence of iron(II)triflate (0.04eq) at 100 °C for 72 hrs. Upon completion, the reaction mixture is cooled to ambient temperature and partitioned between aqueous K₂CO₃ and hexane. The hexane layer is separated, dried over sodium sulphate and evaporated to give a yellow oil, which is distilled to provide the target ether.

Additionally, certain compounds of the disclosure can be prepared according to the scheme below: 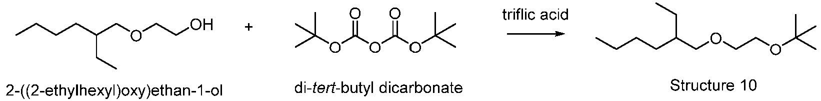 The alcohol, di-tert-butyl-carbonate (4 eq.) and triflic acid (0.025 eq.) are placed in an autoclave and heated to 60 °C for 72 hrs, up to 40 bars of pressure. The autoclave is carefully vented, and the reaction mixture is added to water and extracted with hexane. The hexane layer is dried over sodium sulphate, reduced in vacuum to an oil which is distilled to provide the target diether. Alternatively, triflic acid (0.1 eq.) is added cautiously to a suspension of the alcohol (60 mmol) in MTBE (70 mL) at ambient temperature and the mixture is warmed to 60 °C, followed by the dropwise addition of di-tert-butyl-carbonate (7 eq.) dissolved in MTBE (7 mLI) over 1 hour. The reaction mixture is heated at 60 °C for 18 hrs, cooled to ambient temperature, diluted with hexane, washed with water, dried over sodium sulphate, and reduced in vacuum to an oil which is distilled to provide the target diether.

Compounds of the disclosure include those of the table below:

| | |
|---|---|
| 1 | 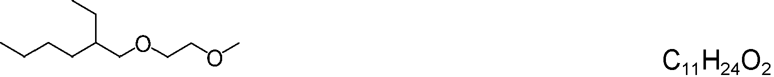 |
| 2 | 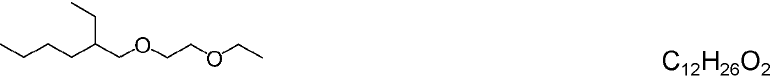 |
| 3 | 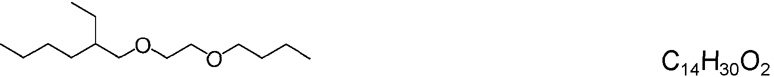 |
| 4 | 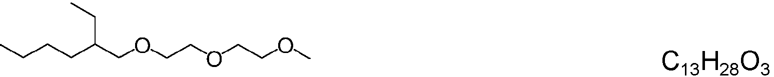 |
| 5 | 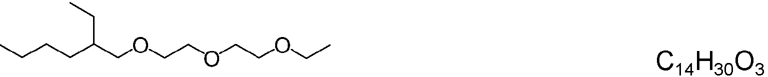 |
| 6 | 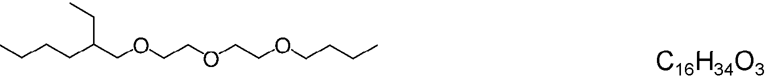 |

| | |
|---|---|
| 7 | 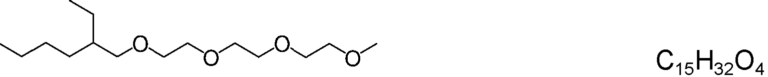 |
| 8 | 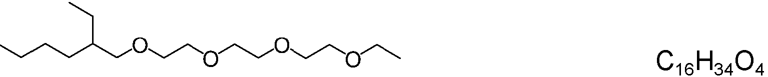 |
| 9 | 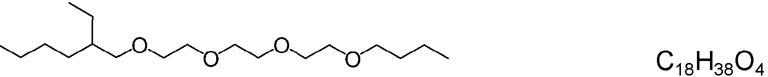 |

| | |
|---|---|
| 10 | 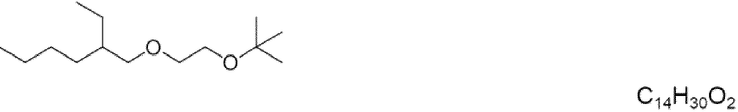 |
| 11 | 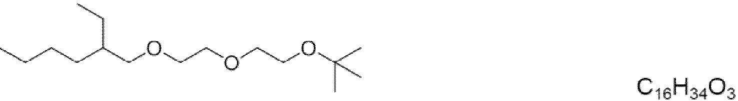 |
| 12 | 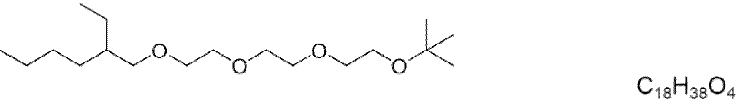 |
| 13 | 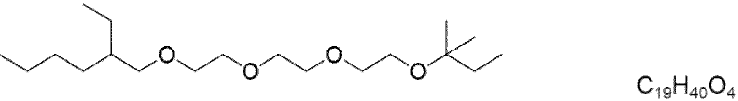 |
| 14 | 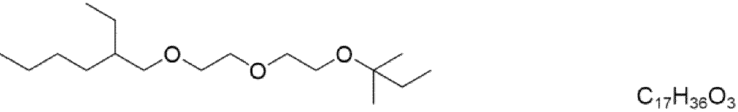 |
| 15 | 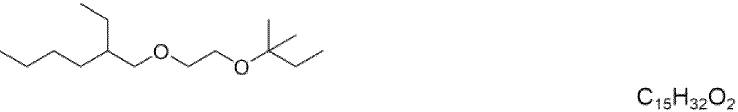 |
| 16 | 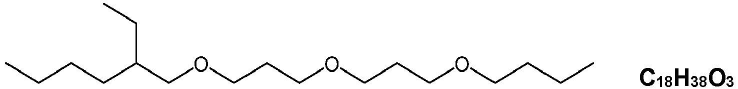 |
| 17 | 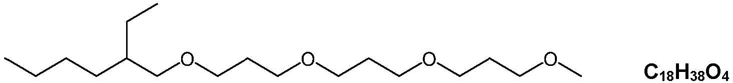 |

The following table presents predictions of properties (kinematic viscosities at 40 °C and 100 °C, viscosity index, pour point, oxidation threshold temperature, flashpoint, for the structures above, based on comparison with the properties of known molecules.

| # | Mol Wt | KV40 | KV100 | PP | DSC ox | Flash Pt |
|---|---|---|---|---|---|---|
| | g/mol | m²/s (cSt) | m²/s (sCt) | °C | °C | °C |
| 1 | 188.311 | 2.75 x10⁻⁶ (2.75) | 4 x10⁻⁷ (0.4) | -95 | 200 | 90 |
| 2 | 202.338 | 2.8 x10⁻⁶ (2.8) | 5 x10⁻⁷ (0.5) | -90 | 200 | 95 |
| 3 | 230.392 | 1.85 x10⁻⁶ (1.85) | 8.4x10⁻⁷ (0.84) | -102 | 200 | 112 |
| 4 | 232.364 | 3 x10⁻⁶ (3) | 6 x10⁻⁷ (0.6) | -95 | 200 | 112 |
| 5 | 246.391 | 2.75 x10⁻⁶ (2.75) | 7.5 x10⁻⁷ (0.75) | -95 | 200 | 120 |
| 6 | 274.445 | 2.67 x10⁻⁶ (2.67) | 1.13 x10⁻⁶ (1.13) | -90 | 200 | 143 |
| 7 | 276.417 | 2.9 x10⁻⁶ (2.9) | 1.2 x10⁻⁶ (1.2) | -90 | 200 | 148.5 |
| 8 | 290.444 | 3.07 x10⁻⁶ (3.07) | 1.26 x10⁻⁶ (1.26) | -81 | 200 | 153.5 |
| 9 | 318.498 | 4 x10⁻⁶ (4) | 1.6 x10⁻⁶ (1.6) | -90 | 200 | 160 |
| 10 | 230.392 | 3 x10⁻⁶ (3) | 5 x10⁻⁷ (0.5) | -90 | 210 | 105 |
| 11 | 274.445 | 3 x10⁻⁶ (3) | 9.5 x10⁻⁷ (0.95) | -96 | 210 | 125 |
| 12 | 318.498 | 4.1 x10⁻⁶ (4.1) | 1.7 x10⁻⁶ (1.7) | -95 | 210 | 155 |
| 13 | 332.525 | 4.1 x10⁻⁶ (4.1) | 1.45 x10⁻⁶ (1.45) | -90 | 210 | 160 |
| 14 | 288.472 | 2.9 x10⁻⁶ (2.9) | 1.15 x10⁻⁶ (1.15) | -95 | 210 | 140 |
| 15 | 244.419 | 2.9 x10⁻⁶ (2.9) | 7.5 x10⁻⁷ (0.75) | -95 | 210 | 110 |
| 16 | 302.499 | 3.01 x10⁻⁶ (3.01) | 1.23 x10⁻⁶ (1.23) | -105 | 210 | 142.4 |
| 17 | 318.498 | 3.23 x10⁻⁶ (3.23) | 1.29 x10⁻⁶ (1.29) | -93 | 210 | 145.4 |

In closing, it is to be understood that the various embodiments herein are illustrative of the methods of the disclosures. Accordingly, the methods of the present disclosure are not limited to that precisely as shown and described.

## Claims

1. A method comprising:
contacting a thermal management fluid with a surface having a temperature of at least 25 °C, the surface being in substantial thermal communication with a heat source; and
absorbing thermal energy in the thermal management fluid from the heat source through the surface;
wherein the thermal management fluid comprises:
one or more dielectric compounds of formula (I): 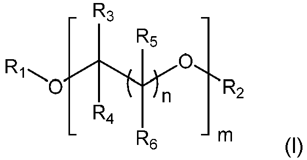 wherein
m is an integer 1, 2, 3, 4 or 5;
n is an integer 1, 2, 3, 4 or 5;
R₁ is C₁-C₅ alkyl;
R₂ is C₆-C₁₂ alkyl;
each R₃ and R₄ are independently selected from H and C₁-C₆ alkyl; and
each R₅ and R₆ are independently selected from H and C₁-C₆ alkyl;
the one or more dielectric compounds being present in a total amount in the range of 1 wt% to 100 wt%, based on the total weight of the thermal management fluid; and
wherein the thermal management fluid has a flash point of at least 100 °C, measured in accordance with ASTM D93, and the thermal management fluid has a dielectric constant of at least 1.5 at 25 °C.

2. The method according to claim 1, wherein the heat source is an electrical component, e.g., a battery system, a capacitor, inverter, electrical cabling, a fuel cell, a motor, a computer, or high power charging equipment.

3. A battery system comprising:
a housing;
one or more electrochemical cells disposed in the housing;
a fluid path extending in the housing and in substantial thermal communication with the one or more electrochemical cells; and
a thermal management fluid disposed in the fluid path;
wherein the thermal management fluid comprises:
one or more dielectric compounds of formula (I): 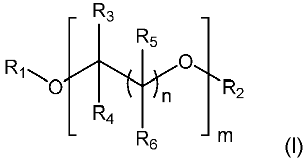 wherein
m is an integer 1, 2, 3, 4 or 5;
n is an integer 1, 2, 3, 4 or 5;
R₁ is C₁-C₅ alkyl;
R₂ is C₆-C₁₂ alkyl;
each R₃ and R₄ are independently selected from H and C₁-C₆ alkyl; and
each R₅ and R₆ are independently selected from H and C₁-C₆ alkyl;
the one or more dielectric compounds being present in a total amount in the range of 1 wt% to 100 wt%, based on the total weight of the thermal management fluid; and
wherein the thermal management fluid has a flash point of at least 100 °C, measured in accordance with ASTM D93, and the thermal management fluid has a dielectric constant of at least 1.5 at 25 °C.

4. A thermal management circuit comprising:
a fluid path extending around and/or through a heat source;
a thermal management fluid disposed in and configured to circulate in the fluid path and to absorb thermal energy produced by the heat source,
wherein the fluid is disposed in the fluid path, the heat exchanger, the pump and the connecting duct;
wherein the thermal management fluid comprises:
one or more dielectric compounds of formula (I): 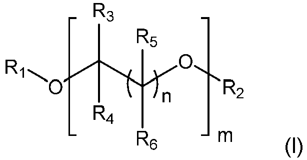 wherein
m is an integer 1, 2, 3, 4 or 5;
n is an integer 1, 2, 3, 4 or 5;
R₁ is C₁-C₅ alkyl;
R₂ is C₆-C₁₂ alkyl;
each R₃ and R₄ are independently selected from H and C₁-C₆ alkyl; and
each R₅ and R₆ are independently selected from H and C₁-C₆ alkyl;
the one or more dielectric compounds being present in a total amount in the range of 1 wt% to 100 wt%, based on the total weight of the thermal management fluid; and
wherein the thermal management fluid has a flash point of at least 100 °C, measured in accordance with ASTM D93, and the thermal management fluid has a dielectric constant of at least 1.5 at 25 °C.

5. The method, battery system or thermal management circuit of any of claims 1-4, wherein R₂ is a branched C₆-C₁₂ alkyl.

6. The method, battery system or thermal management circuit of any of claims 1-5, wherein each of R₃, R₄, R₅ and R₆ is independently selected from H and methyl.

7. The method, battery system or thermal management circuit of any of claims 1-5, wherein each R₄ is H and each R₃ is H or C₁-C₆ alkyl (such as C₁-C₄ alkyl or C₁-C₂ alkyl); and each R₅ is H and each R₆ is H or C₁-C₆ alkyl (such as C₁-C₄ alkyl or C₁-C₂ alkyl).

8. The method, battery system or thermal management circuit of any of claims 1-5, wherein R₃ is H, R₄ is H, R₅ is H and each R₆ is H or C₁-C₆ alkyl (such as C₁-C₄ alkyl or C₁-C₂ alkyl).

9. The method, battery system or thermal management circuit of claims 1-4, wherein the one or more dielectric compounds have the formula 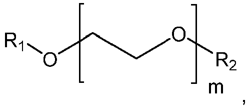 in which m is 1, 2 or 3, R₁ is C₁-C₅ alkyl, and R₂ is C₆-C₁₀ alkyl.

10. The method, battery system or thermal management circuit of claim 9, wherein m is 1, 2 or 3.

11. The method, battery system or thermal management circuit of any of claims 1-10, wherein each of the one or more compounds contains a total number of carbon atoms from 10 to 30.

12. The method, battery system or thermal management circuit of any of claims 1-4, wherein the one or more dielectric compounds are independently selected from: 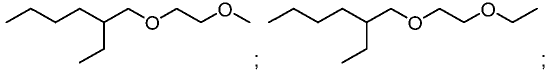 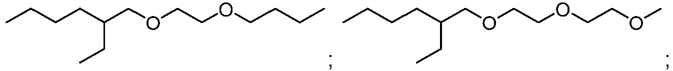 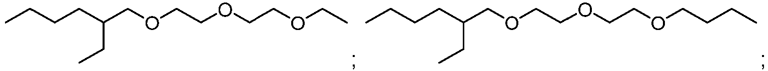 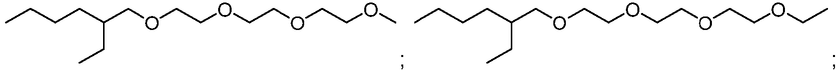 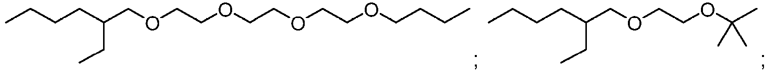 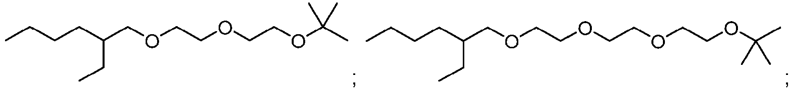 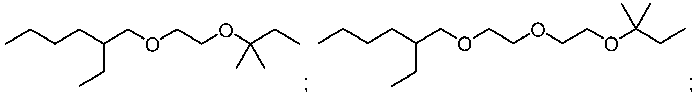 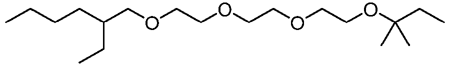 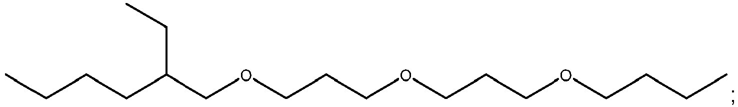 and

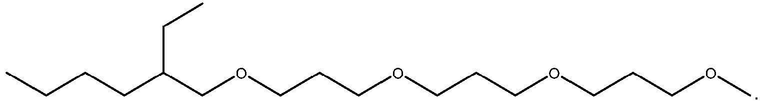

13. The method, battery system or thermal management circuit of any of claims 1-12 wherein the one or more dielectric compounds is present in an amount in the range of 50 wt% to 100 wt%, based on the total weight of the thermal management fluid.

14. The method, battery system or thermal management circuit of any of claims 1-13, wherein the thermal management fluid has a kinematic viscosity at 40 °C in the range of 0.0000015 to 0.00002 m²/s (1.5 to 20 cSt), as measured in accordance with ASTM D455.

15. A thermal management fluid comprising:
one or more dielectric compounds have the formula 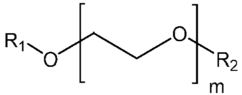 wherein
m is an integer 1, 2, or 3;
R₁ is methyl, ethyl, propyl, or butyl, or
R₁ is isopropyl, t-butyl, sec-butyl, or 1,1-dimethylpropyl; and
R₂ is a group -CH₂-CH(R_{b})(R_{d}) in which R_{b} is methyl or ethyl, and R_{d} is C₃-C₈ alkyl, e.g., R₂ is 2-ethylhexyl;
the one or more dielectric compounds being present in a total amount in the range of 1 wt% to 100 wt%, based on the total weight of the thermal management fluid; and
wherein the thermal management fluid has a flash point of at least 100 °C, measured in accordance with ASTM D93, and the thermal management fluid has a dielectric constant of at least 1.5 at 25 °C.

## Patentansprüche

1. Ein Verfahren, das Folgendes umfasst:
In-Kontakt-Bringen eines Thermomanagementfluids mit einer Oberfläche mit einer Temperatur von mindestens 25 °C, wobei die Oberfläche in wesentlicher thermischer Verbindung mit einer Wärmequelle steht; und
Absorbieren thermischer Energie in dem Thermomanagementfluid von der Wärmequelle durch die Oberfläche;
wobei das Thermomanagementfluid Folgendes umfasst:
eine oder mehrere dielektrische Verbindungen der Formel (I): 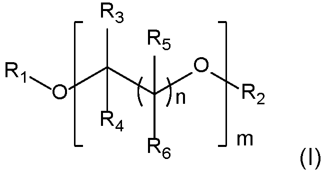 wobei
m eine ganze Zahl 1, 2, 3, 4 oder 5 ist;
n eine ganze Zahl 1, 2, 3, 4 oder 5 ist;
R₁ C₁-C₅-Alkyl ist;
R₂ C₆-C₁₂-Alkyl ist;
jedes R₃ und R₄ unabhängig aus H und C₁-C₆-Alkyl ausgewählt sind; und
jedes R₅ und R₆ unabhängig aus H und C₁-C₆-Alkyl ausgewählt sind;
die eine oder mehreren dielektrischen Verbindungen, bezogen auf das Gesamtgewicht des Thermomanagementfluids, in einer Gesamtmenge im Bereich von 1 Gew.-% bis 100 Gew.-% vorhanden sind; und
wobei das Thermomanagementfluid einen Flammpunkt von mindestens 100 °C, gemessen gemäß ASTM D93, aufweist und das Thermomanagementfluid eine Dielektrizitätskonstante von mindestens 1,5 bei 25 °C aufweist.

2. Verfahren nach Anspruch 1, wobei die Wärmequelle ein elektrisches Bauteil ist, z. B. ein Batteriesystem, ein Kondensator, Wechselrichter, elektrisches Kabel, eine Brennstoffzelle, ein Motor, ein Computer oder Hochleistungsladegerät.

3. Ein Batteriesystem, das Folgendes umfasst
ein Gehäuse;
eine oder mehrere elektrochemische Zellen, die in dem Gehäuse angeordnet sind;
eine Fluidbahn, die sich in dem Gehäuse erstreckt und in wesentlicher thermischer Verbindung mit der einen oder den mehreren elektrochemischen Zellen steht; und
ein Thermomanagementfluid, das in der Fluidbahn angeordnet ist;
wobei das Thermomanagementfluid Folgendes umfasst:
eine oder mehrere dielektrische Verbindungen der Formel (I): 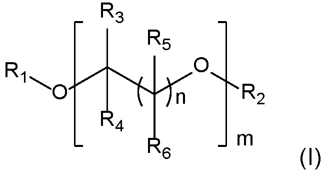 wobei
m eine ganze Zahl 1, 2, 3, 4 oder 5 ist;
n eine ganze Zahl 1, 2, 3, 4 oder 5 ist;
R₁ C₁-C₅-Alkyl ist;
R₂ C₆-C₁₂-Alkyl ist;
jedes R₃ und R₄ unabhängig aus H und C₁-C₆-Alkyl ausgewählt sind; und
jedes R₅ und R₆ unabhängig aus H und C₁-C₆-Alkyl ausgewählt sind;
die eine oder mehreren dielektrischen Verbindungen, bezogen auf das Gesamtgewicht des Thermomanagementfluids, in einer Gesamtmenge im Bereich von 1 Gew.-% bis 100 Gew.-% vorhanden sind; und
wobei das Thermomanagementfluid einen Flammpunkt von mindestens 100 °C, gemessen gemäß ASTM D93, aufweist und das Thermomanagementfluid eine Dielektrizitätskonstante von mindestens 1,5 bei 25 °C aufweist.

4. Ein Thermomanagementkreislauf, der Folgendes umfasst:
eine Fluidbahn, die sich um und/oder durch eine Wärmequelle erstreckt;
ein Thermomanagementfluid, das in der Fluidbahn angeordnet ist und dazu ausgelegt ist, in ihr umzulaufen und thermische Energie zu absorbieren, die von der Wärmequelle produziert wird,
wobei das Fluid in der Fluidbahn, dem Wärmetauscher, der Pumpe und der Verbindungsleitung angeordnet ist;
wobei das Thermomanagementfluid Folgendes umfasst:
eine oder mehrere dielektrische Verbindungen der Formel (I): 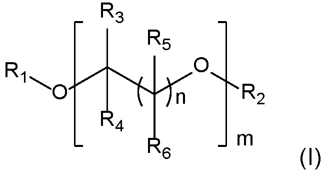 wobei
m eine ganze Zahl 1, 2, 3, 4 oder 5 ist;
n eine ganze Zahl 1, 2, 3, 4 oder 5 ist;
R₁ C₁-C₅-Alkyl ist;
R₂ C₆-C₁₂-Alkyl ist;
jedes R₃ und R₄ unabhängig aus H und C₁-C₆-Alkyl ausgewählt sind; und
jedes R₅ und R₆ unabhängig aus H und C₁-C₆-Alkyl ausgewählt sind;
die eine oder mehreren dielektrischen Verbindungen, bezogen auf das Gesamtgewicht des Thermomanagementfluids, in einer Gesamtmenge im Bereich von 1 Gew.-% bis 100 Gew.-% vorhanden sind; und
wobei das Thermomanagementfluid einen Flammpunkt von mindestens 100 °C, gemessen gemäß ASTM D93, aufweist und das Thermomanagementfluid eine Dielektrizitätskonstante von mindestens 1,5 bei 25 °C aufweist.

5. Verfahren, Batteriesystem oder Thermomanagementkreislauf nach einem der Ansprüche 1-4, wobei R₂ ein verzweigtes C₆-C₁₂-Alkyl ist.

6. Verfahren, Batteriesystem oder Thermomanagementkreislauf nach einem der Ansprüche 1-5, wobei jedes von R₃, R₄, R₅ und R₆ unabhängig aus H und Methyl ausgewählt ist.

7. Verfahren, Batteriesystem oder Thermomanagementkreislauf nach einem der Ansprüche 1-5, wobei jedes R₄ H ist und jedes R₃ H oder C₁-C₆-Alkyl ist (wie etwa C₁-C₄-Alkyl oder C₁-C₂-Alkyl); und jedes R₆ H ist und jedes R₆ H oder C₁-C₆-Alkyl ist (wie etwa C₁-C₄-Alkyl oder C₁-C₂-Alkyl).

8. Verfahren, Batteriesystem oder Thermomanagementkreislauf nach einem der Ansprüche 1-5, wobei R₃ H ist, R₄ H ist, R₅ H ist und jedes R₆ H oder C₁-C₆-Alkyl ist (wie etwa C₁-C₄-Alkyl oder C₁-C₂-Alkyl).

9. Verfahren, Batteriesystem oder Thermomanagementkreislauf nach Ansprüchen 1-4, wobei die eine oder mehreren dielektrischen Verbindungen die Formel 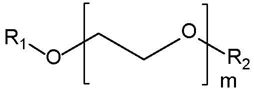 aufweisen, wobei m 1, 2 oder 3 ist, R₁ C₁-C₅-Alkyl ist und R₂ C₆-C₁₀-Alkyl ist.

10. Verfahren, Batteriesystem oder Thermomanagementkreislauf nach Anspruch 9, wobei m 1, 2 oder 3 ist.

11. Verfahren, Batteriesystem oder Thermomanagementkreislauf nach einem der Ansprüche 1-10, wobei jede von der einen oder den mehreren Verbindungen eine Gesamtzahl von Kohlenstoffatomen von 10 bis 30 enthält.

12. Verfahren, Batteriesystem oder Thermomanagementkreislauf nach einem der Ansprüche 1-4, wobei die eine oder mehreren dielektrischen Verbindungen unabhängig ausgewählt sind aus: 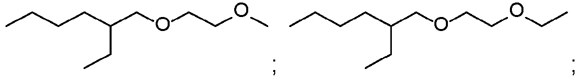 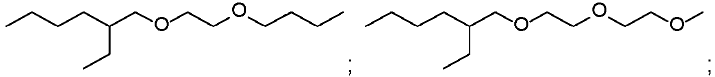 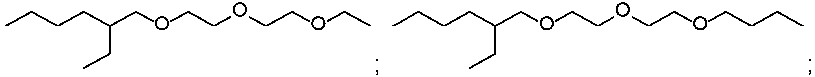 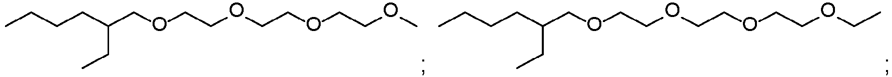 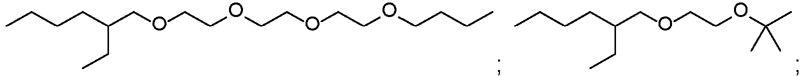 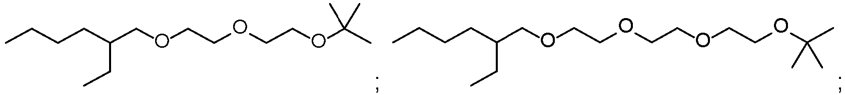 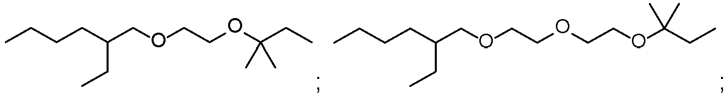 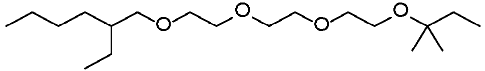 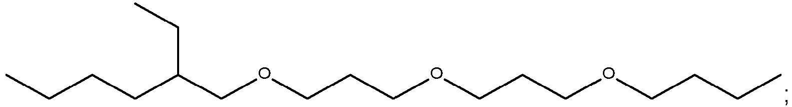 und

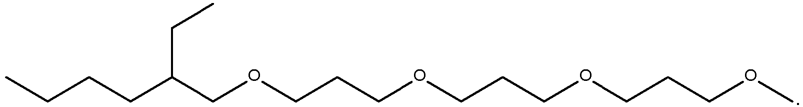

13. Verfahren, Batteriesystem oder Thermomanagementkreislauf nach einem der Ansprüche 1-12, wobei die eine oder mehreren dielektrischen Verbindungen, bezogen auf das Gesamtgewicht des Thermomanagementfluids, in einer Menge im Bereich von 50 Gew.-% bis 100 Gew.-% vorhanden sind.

14. Verfahren, Batteriesystem oder Thermomanagementkreislauf nach einem der Ansprüche 1-13, wobei das Thermomanagementfluid eine kinematische Viskosität bei 40 °C im Bereich von 0,0000015 bis 0,00002 m²/s (1,5 bis 20 cSt), gemessen gemäß ASTM D455, aufweist.

15. Ein Thermomanagementfluid, das Folgendes umfasst:
eine oder mehrere dielektrische Verbindungen mit der Formel 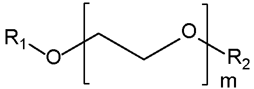 wobei
m eine ganze Zahl 1, 2 oder 3 ist;
R₁ Methyl, Ethyl, Propyl oder Butyl ist oder
R₁ Isopropyl, t-Butyl, sec-Butyl oder 1,1-Dimethylpropyl ist; und
R₂ eine Gruppe -CH₂-CH(R_{b})(R_{d}) ist, wobei R_{b} Methyl oder Ethyl ist und R_{d} C₃-C₈-Alkyl ist, z. B. R₂ 2-Ethylhexyl ist;
die eine oder mehreren dielektrischen Verbindungen, bezogen auf das Gesamtgewicht des Thermomanagementfluids, in einer Gesamtmenge im Bereich von 1 Gew.-% bis 100 Gew.-% vorhanden sind; und
wobei das Thermomanagementfluid einen Flammpunkt von mindestens 100 °C, gemessen gemäß ASTM D93, aufweist und das Thermomanagementfluid eine Dielektrizitätskonstante von mindestens 1,5 bei 25 °C aufweist.

## Revendications

1. Procédé comprenant :
la mise en contact d'un fluide de gestion thermique avec une surface ayant une température d'au moins 25 °C, la surface étant en communication thermique substantielle avec une source de chaleur ; et
l'absorption d'énergie thermique dans le fluide de gestion thermique de la source de chaleur à travers la surface,
dans lequel le fluide de gestion thermique comprend :
un ou plusieurs composés diélectriques de formule (I) : 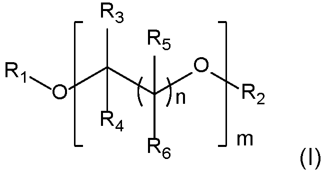 dans lequel
m est un nombre entier 1, 2, 3, 4 ou 5 ;
n est un nombre entier 1, 2, 3, 4 ou 5 ;
R₁ est un alkyle en C₁-C₅ ;
R₂ est un alkyle en C₆-C₁₂ ;
chaque R₃ et R₄ sont indépendamment choisis parmi H et un alkyle en C₁-C₆ ; et
chaque R₅ et R₆ sont indépendamment choisis parmi H et un alkyle en C₁-C₅ ;
les un ou plusieurs composés diélectriques étant présents en une quantité totale dans la plage de 1 % en poids à 100 % en poids, sur la base du poids total du fluide de gestion thermique ; et
dans lequel le fluide de gestion thermique a un point d'éclair d'au moins 100 °C, mesuré conformément à la norme ASTM D93, et le fluide de gestion thermique a une constante diélectrique d'au moins 1,5 à 25 °C.

2. Procédé selon la revendication 1, dans lequel la source de chaleur est un composant électrique, par exemple, un système de batterie, un condensateur, un inverseur, un câblage électrique, une pile à combustible, un moteur, un ordinateur ou un équipement de charge à haute puissance.

3. Système de batterie comprenant :
un logement ;
une ou plusieurs cellules électrochimiques disposées dans le logement ;
un chemin de fluide s'étendant dans le logement et en communication thermique substantielle avec les une ou plusieurs cellules électrochimiques ; et
un fluide de gestion thermique disposé dans le chemin de fluide :
dans lequel le fluide de gestion thermique comprend :
un ou plusieurs composés diélectriques de formule (I) : 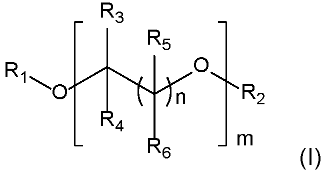 dans lequel
m est un nombre entier 1, 2, 3, 4 ou 5 ;
n est un nombre entier 1, 2, 3, 4 ou 5 ;
R₁ est un alkyle en C₁-C₅ ;
R₂ est un alkyle en C₆-C₁₂ ;
chaque R₃ et R₄ sont indépendamment choisis parmi H et un alkyle en C₁-C₆ ; et
chaque R₅ et R₆ sont indépendamment choisis parmi H et un alkyle en C₁-C₅ ;
les un ou plusieurs composés diélectriques étant présents en une quantité totale dans la plage de 1 % en poids à 100 % en poids, sur la base du poids total du fluide de gestion thermique ; et
dans lequel le fluide de gestion thermique a un point d'éclair d'au moins 100 °C, mesuré conformément à la norme ASTM D93, et le fluide de gestion thermique a une constante diélectrique d'au moins 1,5 à 25 °C.

4. Circuit de gestion thermique comprenant :
un chemin de fluide s'étendant autour et/ou à travers une source de chaleur ;
un fluide de gestion thermique disposé dans et configuré pour circuler dans le chemin de fluide et pour absorber de l'énergie thermique produite par la source de chaleur,
dans lequel le fluide est disposé dans le chemin de fluide, l'échangeur de chaleur, la pompe et le conduit de raccordement ;
dans lequel le fluide de gestion thermique comprend :
un ou plusieurs composés diélectriques de formule (I) : 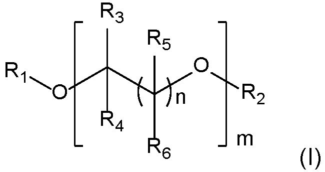 dans lequel
m est un nombre entier 1, 2, 3, 4 ou 5 ;
n est un nombre entier 1, 2, 3, 4 ou 5 ;
R₁ est un alkyle en C₁-C₅ ;
R₂ est un alkyle en C₆-C₁₂ ;
chaque R₃ et R₄ sont indépendamment choisis parmi H et un alkyle en C₁-C₆ ; et
chaque R₅ et R₆ sont indépendamment choisis parmi H et un alkyle en C₁-C₅ ;
les un ou plusieurs composés diélectriques étant présents en une quantité totale dans la plage de 1 % en poids à 100 % en poids, sur la base du poids total du fluide de gestion thermique ; et
dans lequel le fluide de gestion thermique a un point d'éclair d'au moins 100 °C, mesuré conformément à la norme ASTM D93, et le fluide de gestion thermique a une constante diélectrique d'au moins 1,5 à 25 °C.

5. Procédé, système de batterie ou circuit de gestion thermique selon l'une quelconque des revendications 1 à 4, dans lequel R₂ est un alkyle ramifié en C₆-C₁₂.

6. Procédé, système de batterie ou circuit de gestion thermique selon l'une quelconque des revendications 1 à 5, dans lequel chacun de R₃, R₄, R₅ et R₆ est indépendamment choisi parmi H et un méthyle.

7. Procédé, système de batterie ou circuit de gestion thermique selon l'une quelconque des revendications 1 à 5, dans lequel chaque R₄ est H et chaque R₃ est H ou un alkyle en C₁-C₆ (tel qu'un alkyle en C₁-C₄ ou un alkyle en C₁-C₂) ; et chaque R₅ est H et chaque R₆ est H ou un alkyle en C₁-C₆ (tel qu'un alkyle en C₁-C₄ ou un alkyle en C₁-C₂).

8. Procédé, système de batterie ou circuit de gestion thermique selon l'une quelconque des revendications 1 à 5, dans lequel R₃ est H, R₄ est H, R₅ est H et chaque R₆ est H ou un alkyle en C₁-C₆ (tel qu'un alkyle en C₁-C₄ ou un alkyle en C₁-C₂).

9. Procédé, système de batterie ou circuit de gestion thermique selon les revendications 1 à 4, dans lequel les un ou plusieurs composés diélectriques ont la formule 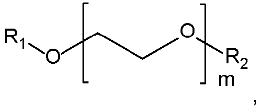 dans laquelle m est 1, 2 ou 3, R₁ est un alkyle en C₁-C₅ et R₂ est un alkyle en C₆-C₁₀.

10. Procédé, système de batterie ou circuit de gestion thermique selon la revendication 9, dans lequel m est 1, 2 ou 3.

11. Procédé, système de batterie ou circuit de gestion thermique selon l'une quelconque des revendications 1 à 10, dans lequel chacun des un ou plusieurs composés contient un nombre total d'atomes de carbone allant de 10 à 30.

12. Procédé, système de batterie ou circuit de gestion thermique selon l'une quelconque des revendications 1 à 14, dans lequel les un ou plusieurs composés diélectriques sont indépendamment choisis parmi : 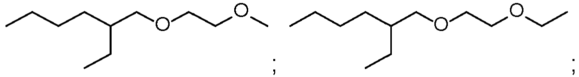 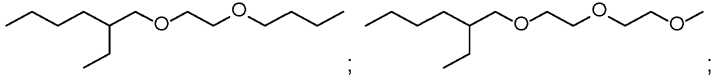 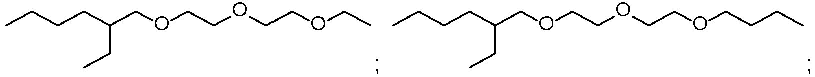 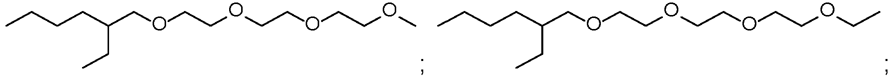 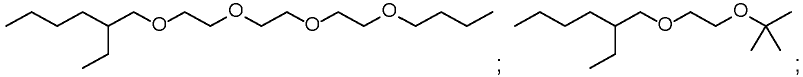 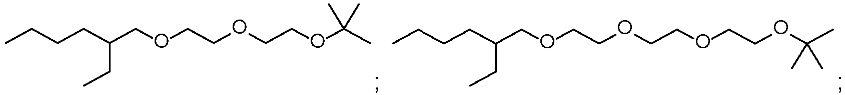 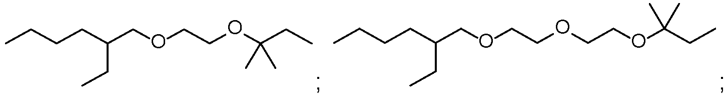 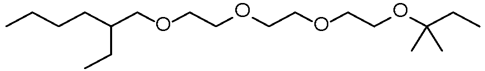 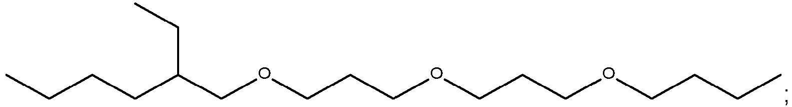 et

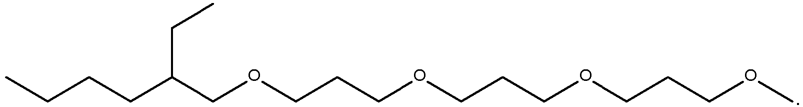

13. Procédé, système de batterie ou circuit de gestion thermique selon l'une quelconque des revendications 1 à 12, dans lequel les un ou plusieurs composés diélectriques sont présents en une quantité dans la plage de 50 % en poids à 100 % en poids, par rapport au poids total du fluide de gestion thermique.

14. Procédé, système de batterie ou circuit de gestion thermique selon l'une quelconque des revendications 1 à 13, dans lequel le fluide de gestion thermique a une viscosité cinématique à 40 °C dans la plage de 0,0000015 à 0,00002 m²/s (1,5 à 20 cSt), telle que mesurée conformément à la norme ASTM D455.

15. Fluide de gestion thermique comprenant :
un ou plusieurs composés diélectriques ont la formule 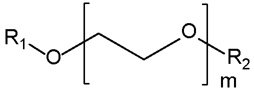 dans lequel
m est un nombre entier 1, 2, ou 3 ;
R₁ est un méthyle, un éthyle, un propyle ou un butyle, ou
R₁ est un isopropyle, un t-butyle, un sec-butyle ou un 1,1-diméthylpropyle ; et
R₂ est un groupe -CH₂-CH(R_{b})(R_{d}) dans lequel R_{b} est un méthyle ou un éthyle, et R_{d} est un alkyle en C₃-C₈, par exemple, R₂ est un 2-éthylhexyle ;
les un ou plusieurs composés diélectriques étant présents en une quantité totale dans la plage de 1 % en poids à 100 % en poids, sur la base du poids total du fluide de gestion thermique ; et
dans lequel le fluide de gestion thermique a un point d'éclair d'au moins 100 °C, mesuré conformément à la norme ASTM D93, et le fluide de gestion thermique a une constante diélectrique d'au moins 1,5 à 25 °C.
